(19)

**Europäisches Patentamt**
**European Patent Office**
**Office européen des brevets**

(11)  **EP 3 718 418 B1**

(12)  # EUROPEAN PATENT SPECIFICATION

(45) Date of publication and mention
of the grant of the patent:
**09.10.2024  Bulletin 2024/41**

(21) Application number: **20169772.9**

(22) Date of filing: **06.08.2015**

(51) International Patent Classification (IPC):
**A23L 33/15** (2016.01)     **A23L 33/10** (2016.01)
**A23C 9/20** (2006.01)     **A61K 31/525** (2006.01)
**A61K 31/7004** (2006.01)     **A61K 35/744** (2015.01)
**A61K 35/745** (2015.01)     **A61K 35/747** (2015.01)
**A61P 3/10** (2006.01)

(52) Cooperative Patent Classification (CPC):
(C-Sets available)
**A23L 33/15; A23L 33/105; A23L 33/135;**
**A61K 31/4415; A61K 31/525; A61K 31/593;**
**A61K 31/7004; A61K 31/714; A61K 35/744;**
**A61K 35/745; A61K 35/747; A61P 3/10;**
**A61P 15/00;** A23V 2002/00; A23V 2200/3204;

(Cont.)

(54) **VITAMIN B2 AND MYO-INOSITOL FOR TREATING AND PREVENTING GESTATIONAL DIABETES**

VITAMIN B2 UND MYO-INOSIT ZUR BEHANDLUNG UND VORBEUGUNG VON
GESTATIONSDIABETES

VITAMINE B2 ET MYO-INOSITOL POUR LE TRAITEMENT ET LA PRÉVENTION DU DIABÈTE
GESTATIONNEL

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **08.08.2014  EP 14180396**
        **08.08.2014  EP 14180401**
        **08.08.2014  EP 14180403**
        **05.06.2015  EP 15170911**
        **05.06.2015  EP 15170915**

(43) Date of publication of application:
**07.10.2020  Bulletin 2020/41**

(62) Document number(s) of the earlier application(s) in
accordance with Art. 76 EPC:
**15750349.1 / 3 177 151**

(73) Proprietor: **Société des Produits Nestlé S.A.**
**1800 Vevey (CH)**

(72) Inventors:
• **SILVA ZOLEZZI, Irma**
  **276750 Singapore (SG)**
• **MACE, Catherine**
  **1093 LA CONVERSION (CH)**

• **GODFREY, Keith Malcolm**
  **Ashurst, Hampshire SO40 7AP (GB)**
• **BAKER, Philip Newton**
  **LEICESTER, LE7 9GE (GB)**
• **CHONG, Yap Seng**
  **268431 Singapore (SG)**

(74) Representative: **Gagliardi, Tatiana**
**Société des Produits Nestlé S.A.**
**Avenue Nestlé 55**
**1800 Vevey (CH)**

(56) References cited:
**EP-A1- 1 932 542        WO-A1-01/43571**
**US-A1- 2002 155 163    US-A1- 2004 072 794**
**US-A1- 2011 028 434    US-A1- 2013 266 687**
**US-A1- 2014 127 166    US-A1- 2014 161 878**

• **BAKER L ET AL: "Diabetic embryopathy: A**
**selective review of recent trends", JOURNAL OF**
**DIABETES AND ITS COMPLICATIONS,**
**ELSEVIER SCIENCE, NEW YORK, NY, US, vol. 7,**
**no. 3, 1 July 1993 (1993-07-01), pages 204 - 212,**
**XP026277613, ISSN: 1056-8727, [retrieved on**
**19930701], DOI: 10.1016/1056-8727(93)90046-2**

(52) Cooperative Patent Classification (CPC): (Cont.)
A23V 2200/328; A23V 2250/641; A23V 2250/7044

C-Sets
**A61K 31/4415, A61K 2300/00;**
**A61K 31/525, A61K 2300/00;**
**A61K 31/593, A61K 2300/00;**
**A61K 31/7004, A61K 2300/00;**
**A61K 31/714, A61K 2300/00;**
**A61K 35/744, A61K 2300/00;**
**A61K 35/745, A61K 2300/00;**
**A61K 35/747, A61K 2300/00;**

A23V 2002/00, A23V 2200/3204, A23V 2200/328,
A23V 2250/641, A23V 2250/7044

**Description**

**Field of the invention**

The present invention relates to a maternal nutrition composition comprising myo-inositol and vitamin B2. Such a composition has been specifically designed to provide optimized nutrition to a woman desiring to get pregnant, to a pregnant woman and/or to a lactating woman. The present invention also relates to the use of vitamin B2, or a composition comprising vitamin B2, to treat or prevent gestational diabetes mellitus and conditions associated therewith in a pregnant subject and/or its offspring.

**Background of the invention**

Scientific evidence has accumulated showing that prenatal and post natal early nutrition and other environmental factors cause programming of long-term health and well-being, and can impact the risk of developing chronic diseases. Several studies have shown that changes in dietary intake or manipulation of individual macro and micronutrients during the reproductive period can have an impact in several physiological processes, such as growth, metabolism, appetite, cardiovascular function among others (Koletzko B et al (2011) Am J Nutr 94(s):2036-43S). Therefore nutritional status (nutrient stores and dietary intake) of women before and during pregnancy is of relevance to optimize neonatal and child health outcomes. Maternal nutrition is thought to affect the availability and supply of nutrients to the developing fetus that are required for critical developmental processes. Nutritional status of the woman before she gets pregnant also has an impact on her health and wellbeing, as well as on the health, growth and well-being of her baby. For example, micronutrient deficiencies have profound and often persistent effects on fetal tissues and organs, even in the absence of clinical signs of their deficiency in the mother (Ashworth CJ et al (2001) 122:527-35). Inadequate intakes of multiple micronutrients are common among women of reproductive age living in resource poor-settings (Torhem LE et al. (2010) J. Nutr. 140: 2051S-58S. Nutritional status of the lactating woman after delivery also has an impact on her health and wellbeing, as well as on the health, growth and well-being of her baby.

A large variety of nutrients have already been used in compositions for maternal administration and various nutritional compositions have been developed to address maternal nutrition needs. These typically contain vitamin and mineral mixes. The protective role of myo-inositol on diabetic embryopathy has been described in e.g:, Baker L, Piddington R; J. Diab. Comp.1993; 7: 204-212 (XP026277613). The use of probiotic bacteria in the manufacture of nutritional supplements or special dietary foods for pregnant women has been proposed in e.g., US 2014/127166 A1. However it would still be useful to specifically target the nutritional deficiencies of this specific population by selecting the most useful nutrients for these women such as to provide compositions tailored to the nutritional needs of women in the reproductive period to optimize the prenatal and postnatal nutrition of mothers, for the benefit of the mother and of her infant. The inventors have also surprisingly found that vitamin B2 may be used to enhance glucose uptake in mammalian cells, in particular in mammalian muscle cells. Accordingly, administering vitamin B2 to a subject before and/or during pregnancy may prevent and/or treat GDM and/or prevent a condition associated with GDM in a pregnant subject or its offspring. The inventors have also found that heterozygous db/db mice when administered a supplemental vitamin B2 during pregnancy have a lower fasting glucose concentration in comparison to mice not administered a supplemental dose. Further, the inventors have found that mice administered myo-inositol in combination with supplemental vitamin B2 have a surprisingly low fasting glucose concentration during pregnancy and pre pregnancy.

Also, it was found that supplementation with B2 resulted in a lower %glucose increase during a first 30 min of OGTT, and that when the B2 was combined with myoinositol, the %glucose increase was even lower.

Further to the above, the inventors have found that heterozygous db/db mice when administered myo-inositol in combination with supplemental vitamin B2 during pregnancy have a surprisingly lower retroperitoneal fat deposition.

As taught in in Athukorala C et al. BMC Pregnancy Childbirth, 2010, 10: 56; Ovesen P et al. Obstet Gynecol, 2011, 118: 305 and Aisling MM rat al. 2009, Diabetes Care 32(7): 1308), there is a well-established link between body fat deposition, obesity and GDM in pregnancy.

During pregnancy hormonal changes occur in a mother's body leading to increased insulin resistance and a higher glucose plasma concentration. These changes help ensure the transfer of nutrients from the mother to the fetus, thereby helping to ensure its optimal growth and development. Ordinarily the increased insulin resistance and higher glucose plasma concentrations are counteracted by increased insulin production by the mother. However, some mothers are not able to produce adequate amounts of insulin to counteract the changes and this can result in Gestational Diabetes Mellitus (hereinafter GDM) in said mothers.

GDM is defined as any degree of glucose intolerance with onset or first recognition during pregnancy (Metzger BE, Coustan DR (Eds.): Proceedings of the Fourth International Work-shop-Conference on Gestational Diabetes Mellitus. Diabetes Care 21(Suppl. 2):B1- B167, 1998).

GDM is a pregnancy disorder that can increase the risk of a number of maternal-fetal conditions, including macrosomia, birth injury, shoulder dystocia, premature delivery, and caesarian delivery (hereinafter C-section). Mothers suffering from GDM also have an increased risk of developing type II diabetes immediately after pregnancy and later in life. Also, the offspring of mothers suffering from GDM have an increased risk of developing an impaired glucose tolerance and/or suffering from excess weight/adiposity and associated metabolic disorders e.g. type II diabetes and obesity.

Accordingly, there is a need to find ways to treat and/or prevent GDM in pregnant subjects.

## Summary of the Invention

The invention is set out in the claims. As stated above, the present inventors have now discovered that a composition for maternal administration comprising myo-inositol together with vitamin B2 is of particular usefulness to address the specific nutritional needs of women desiring to get pregnant, of pregnant and lactating women and of their offspring. Such a composition is therefore an object of the present invention. A further object is the use of said composition to treat or prevent GDM in a pregnant subject e.g. A pregnant woman, cat or dog. As also stated above, it was found that vitamin B2 may be used to enhance glucose uptake in mammalian cells, that heterozygous db/db mice when administered a supplemental vitamin B2 during a preconception and a pregnancy period have a lower fasting glucose concentration in comparison to mice not administered a supplemental dose, and therefore may be used to treat or prevent GDM in a pregnant subject e.g. A pregnant woman, cat or dog.

As stated hereinabove, GDM is associated with a variety of conditions affecting the pregnant subject and/or its offspring e.g. preterm and caesarian delivery, birth injury to the mother or baby, shoulder dystocia, macrosomia, excessive offspring blood glucose concentration, excess weigh/adiposity and associated metabolic disorders e.g. type II diabetes, fatty liver disease and obesity immediately after birth and later in the life of the offspring, and an increased risk for the mother of having or developing type 2 diabetes immediately after birth and later in life. Accordingly, by treating or preventing GDM vitamin B2 may also be used to treat or prevent these conditions in these pregnant subjects or their offspring.

Vitamin B2 may be particularly effective, at treating or preventing GDM in a pregnant subject and/or its offspring, when used in combination with myo-inositol (cis-1,2,3,5-trans-4,6-cyclohexanehexol) and/or one or more probiotic, and/or one or more of vitamin B6, B12 and D and/or zinc. In particularly vitamin B2 may be particularly effective when used in combination with myo- inositol, vitamin D or vitamin D and zinc.

The vitamin B2 and optionally myo-inositol and/or one or more probiotics and/or one or more of vitamin B6, B12 and D and/ or zinc, may be administered enterally to a subject before and/or during pregnancy and/or during lactation.

The vitamin B2, and optionally myo-inositol and/or one or more probiotics and/or one or more of vitamin B6, B12 and D and/ or zinc may be administered or employed in any form suitable for ingestion by a subject e.g. in the form of a powdered nutritional composition to be reconstituted in for example milk, juice or water, a food product, a drink, a nutritional supplement such as a powdered nutritional supplement to be sprinkled on food or dissolved in an aqueous medium for example milk, juice or water, or a nutraceutical.

Vitamin B2, optionally also in combination myo-inositol and/or one or more probiotics and/or one or more of vitamin B6, B12 and D and/ or zinc may be used in the manufacture of a medicament for use to treat or prevent GDM in a pregnant subject.

Vitamin B2, optionally also in combination myo-inositol and/or one or more probiotics and/or one or more of vitamin B6, B12 and D and/or zinc, may be provided along with a label indicating dosage requirements in a kit for use to treat or prevent GDM in a pregnant subject.

## Drawings

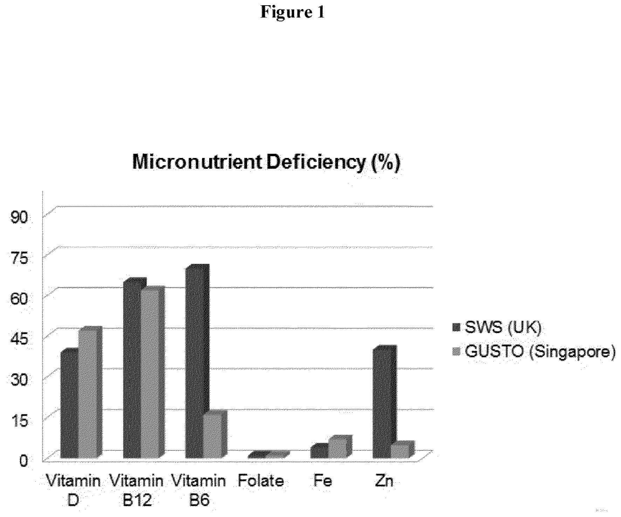

FIG. 1 is a graph showing the percentage of the surveyed women from example 3 found to have deficiencies in vitamin D, vitamin B12, vitamin B6, folate, iron and Zinc respectively.

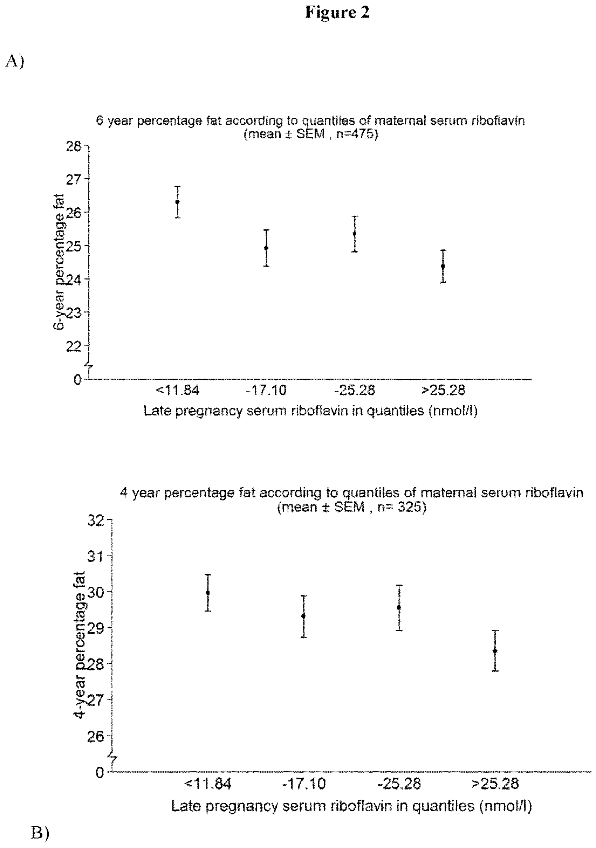

FIG. 2A shows the correlation between low vitamin B2 levels in mothers during pregnancy and the fat mass at 6 years of age of their offspring.

FIG. 2B shows the correlation between low vitamin B2 levels in mothers during pregnancy and the fat mass at 4 years of age of their offspring.

FIG. 3 - Charts illustrating the effect of vitamin B2, in the absence of insulin, on glucose uptake in L6 differentiated muscle cells after 2 hour and 4 hour incubations.

FIG. 4 - Chart illustrating the effect of a combination of vitamin B2 and 1,25-dihydroxyvitamin D3, in the absence of insulin, on glucose uptake in L6 differentiated muscle cells after 2 hour and 4 hour incubations.

FIG. 5 - Chart illustrating the effect of the combination of vitamin B2 and 1,25-dihydroxyvitamin D3 and zinc, in the absence of insulin, on glucose uptake in L6 differentiated muscle cells after a 2 hour incubation.

FIG. 6 - Chart illustrating the fasting blood glucose level of mice, administered myo-inositol, vitamin B2 or a combination thereof, at the end of gestational day (day 18.5 of pregnancy).

FIG. 7 - Chart illustrating the weight of the retroperitoneal fat pads of mice, administered myo-inositol, vitamin B2 or a combination thereof, at the end of gestational day (day 18.5 of pregnancy).

FIG. 8 - Chart illustrating the difference in the OGTT response between WT and heterozygous Lepr db/+ female mice at 4 weeks of age

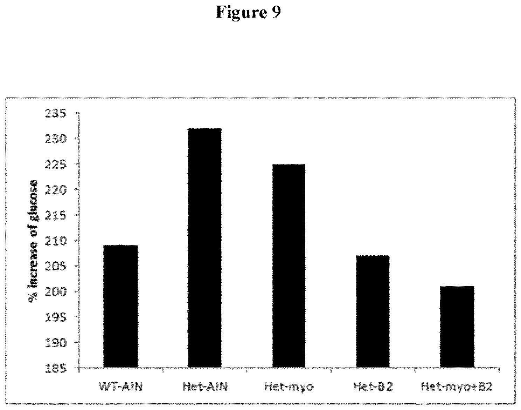

FIG. 9 - Chart illustrating the % increase of glucose from fasting blood glucose level in mice administered myo-inositol, vitamin B2 or a combination thereof, during pregnancy (day 16.5 of pregnancy).

FIG.10 - Chart illustrating the effect of vitamin B2 on glucose uptake in the absence of insulin in insulin resistant L6 differentiated muscle cells after short (2hrs) and long (24hrs) incubation.

FIG. 11 is a chart illustrating the effect of a combination of myo-inositol and probiotics on the delta % fat mass of Goto Kakizaki rats after 10 weeks.

FIG. 12 is a chart illustrating the effect of a combination of myo-inositol and probiotics on the ratio of fat gain to weight gain in Goto Kakizaki rats after 10 weeks.

FIG. 13 is a chart illustrating the effect of a combination of myo-inositol and probiotics on the amount of insulin in the pancreas at day 19.5 of gestation in Goto Kakizaki rats.

FIG. 14 is a chart illustrating the effect of a combination of myo-inositol and probiotics on the AUC insulin increase at day 16.5 of gestation in Goto Kakizaki rats.

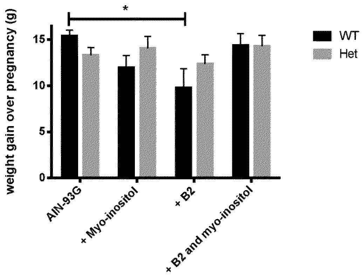

FIG. 15 is a chart illustrating the effect of myo-inositol and vitamin B2 on weight gain during pregnancy in Lepr db/+ mice.

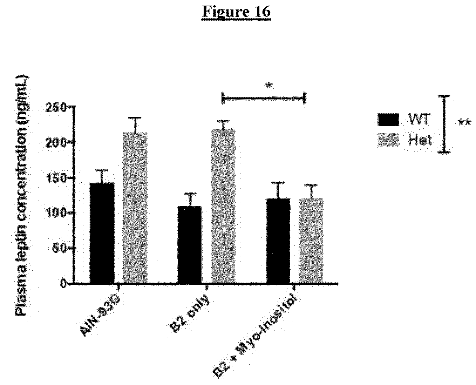

FIG. 16 is a chart illustrating the effect of myo-inositol and vitamin B2 on fasting plasma leptin concentrations at gestational day 18.5 in mice.

## Detailed Description

The present inventors have investigated the specific needs of women desiring to get pregnant, of pregnant and of lactating women to identify the most useful nutrients for this specific population. Based on the studies they have designed an optimized composition comprising myo-inositol and vitamin B2 to specifically address the most acute needs of this specific population.

The composition of the invention is a composition for maternal administration comprising myo-inositol and vitamin B2, and comprising vitamin B6, vitamin B12, vitamin D, Bifidobacterium lactis BB12 deposited as CNCM I-3446, and Lactobacillus rhamnosus GG available under the deposit number CGMCC 1.3724.

The present invention also relates to a kit of parts comprising the ingredients of the composition of the invention, comprising at least two physically separated compositions each comprising one of the ingredients of the composition of the invention, characterized in that at least one of the physically separated compositions comprises myo-inositol and at least one of the physically separated compositions comprises vitamin B2.

Inositol or cyclohexane-1,2,3,4,5,6-hexol is a polyol existing under nine stereoisomeric forms depending on the spatial orientation of its six hydroxyl groups. Myo-Inositol, or cis-1,2,3,5-trans-4,6-cyclohexanehexol, is the predominant isomeric form of inositol. Myo-Inositol is a compound present in animal and plant cells and plays an important role in various cellular processes, as the structural basis for secondary messengers in eukaryotic cells, in particular as inositol triphosphates (IP3), phosphatidylinositol phosphate lipids (PIP2/PIP3) and inositol glycans. Myo-inositol has been shown to participate in a variety of biological process such as cell growth and survival, development and function of peripheral nerves, osteogenesis, energy metabolism and reproduction (Croze et al. (2013) Biochimie 95:1811-1827). Myo-inositol is found as free-from, phosphoinositides and phytic acid, in fresh fruits and vegetables, and in all foods containing seeds (beans, grains and nuts) (Clements RS and Darnell B. Am J Clin Nutr (1980) 33:1954-1967). Myo-Inositol from phytic acid can be released in the gut by the enzymes phytases found in plants, microorganisms and in animal tissues (Schlemmer U et al. Mol Nutr Food Res (2009) 53:S330-S375). These enzymes are capable of releasing free inositol, orthophosphate, and intermediary products including the mono-, di-, tri-, tetra- and penta-phosphate forms of inositol. Much of the ingested inositol hexaphosphate is hydrolysed to inositol.

The term myo-inositol as used herein refers to myo-inositol (cis-1,2,3,5-trans-4,6-cyclohexanehexol) and /or a metabolite thereof.

The term metabolite as used herein refers to any substance produced and/or formed by the body of a subject from a particular compound after its administration e.g. ingestion. It includes active forms and catabolites of a compound.

Any source of myo-inositol suitable for ingestion by the pregnant subject may be used in the invention.

A metabolite of myo-inositol can be selected from the group consisting of D-chiro-inositol, L-chiro-inositol and a combination of the foregoing. In particular the metabolite is D-chiro-inositol.

Due to its important physiological role, and in particular due to its ability to promote cellular growth, myo-inositol is of particular interest for women desiring to get pregnant and for pregnant and lactating women. However, the dietary intake study carried out by the inventors and detailed in Example 4 has shown that pregnant women usually do not take enough of the kind of food in which myo-inositol is found, mostly fruits and vegetables. The present inventors have therefore found that it is of particular benefit for women desiring to get pregnant, for pregnant women and for lactating women to get supplementation in myo-inositol.

Vitamin B2 is beneficial both for the mother and the offspring when administered to pregnant women. In particular the present inventors have recently identified the effect of maternal administration of vitamin B2 for the prevention of increased adiposity, overweight or obesity in the offspring (unpublished patent application EP 14161190.5 and Example 5 of the present application). The present inventors have now carried out a study detailed in Example 4, which demonstrated that a high proportion of pregnant women did not ingest a sufficient amount of vitamin B2 in their normal diet. It is therefore of particular interest to supplement the diet of pregnant women with this vitamin.

The term vitamin B2 as used herein refers to vitamin B2 and /or a metabolite thereof.

Any source of Vitamin B2 suitable for ingestion in the pregnant subject may be used. In particular, vitamin B2 may be riboflavin e.g. riboflavin sold under the trademark Riboflavin Universal.

A metabolite of vitamin B2 can be selected from the group consisting of flavin mononucleotide (hereinafter FMN), Flavin Adenine Dinucleotide (hereinafter FAD), and salts thereof e.g. riboflavin-5'-phosphate sodium salt.

The myo-inositol is preferably provided in an amount of 0.2 to 5 g, preferably 1.5 to 5 g, more preferably 2 to 5 g, most preferably 2 to 4 g per daily dose. The vitamin B2 is preferably provided in an amount of from 0.14 to 14 mg per daily dose.

In a preferred embodiment, the composition of the invention further comprises at least one additional vitamin selected from vitamin D, vitamin B6, vitamin B12 and mixtures thereof. Preferably the composition comprises vitamin D, vitamin B6 and vitamin B12.

The term vitamin B12 as used herein refers to vitamin B12 and /or a metabolite thereof.

The term vitamin B6 as used herein refers to vitamin B6 and /or a metabolite thereof.

The term vitamin D as used herein refers to vitamin D, a precursor thereof, and /or a metabolite thereof.

The term precursor as used herein refers to any substance administered e.g. ingested by a subject and used by the body of said subject to produce and/or form a particular compound.

In particular the vitamin B6 may be pyroxidine hydrochloride, and/or a metabolite of vitamin B6 selected from the group consisting of Pyridoxal 5'-phosphate (hereinafter PLP).

In particular the vitamin B12 may be cyanocobalamin, and/or a metabolite of vitamin B12 selected from the group consisting of hydroxocobalamin, methylcobalamin, adenosylcobalamin, and a combination thereof.

In particular, the vitamin D may be vitamin D2, vitamin D3 or a combination thereof, a precursor of vitamin D selected from the group consisting of 7-dehydrocholecalciferol, a metabolite of vitamin D selected from the group consisting of 25-hydroxyvitamin D3, 1,25-dihydroxyvitamin D3, 25-hydroxyvitamin D2, 1,25-dihydroxyvitamin D2, and a combination of the foregoing.

Most preferably, the vitamin D is vitamin D3, a metabolite of vitamin D selected from the group consisting of 25-hydroxyvitamin D3, 1,25-dihydroxyvitamin, and combination of any of the foregoing. Even more particularly the vitamin D3 is cholecalciferol.

If present the vitamin B6 is preferably provided in an amount of from 0.19 to 19 mg per daily dose. If present, the vitamin B12 is preferably provided in an amount of from 0.26 to 26 $\mu$g per daily dose. If present, the vitamin D is preferably provided in an amount of from 1.5 to 100 $\mu$g per daily dose.

Most preferably the composition of the invention comprises 1.8 mg of vitamin B2, 2.6 mg of vitamin B6, 5.2 $\mu$g of vitamin B12 and 10 $\mu$g of vitamin D per daily dose.

In the study detailed in Example 3, the present inventors have surprisingly shown that pregnant women were more often deficient in vitamins B6, B12 and D compared to other nutrients. It is therefore of particular interest to supplement the diet of pregnant women with these vitamins in order to compensate these particularly often-occurring deficiencies.

The study of Example 3 has also provided evidence of common zinc deficiencies in at least certain populations of pregnant women, even though in a lesser extent than vitamins B2, B12, B6 and D. It is therefore also particularly advantageous to supplement the diet of pregnant women with zinc. Thus, in a preferred embodiment the composition of the invention further comprises zinc. More preferably zinc is present in an amount of from 1.1 to 40 mg per daily dose.

Any source or form of zinc suitable for ingestion by the pregnant subject may be used. In particular the zinc may be a zinc salt such as zinc chloride, zinc picolinate, zinc sulfate, zinc oxide, zinc acetate, zinc carbonate, and combinations of the foregoing.

More particularly the zinc is zinc chloride.

In another preferred embodiment, the composition further comprises probiotics, as these have been found to improve the gut barrier function and to help nutrients pass through the gut (Cani PD et al. (2009) Gut 58:1091-1103). Combining myo-inositol and vitamin B2 with probiotics thus enhances the absorption of these nutrients and other nutrients that may be present in the composition.

The term probiotic as used herein refers to live probiotic bacteria, non-replicating probiotic bacteria, dead probiotic bacteria, non-viable probiotic bacteria, fragments of probiotic bacteria such as DNA, metabolites of probiotic bacteria, cytoplasmic compounds of probiotic bacteria, cell wall materials of probiotic bacteria, culture supernatants of probiotic bacteria, and combinations of any of the foregoing.

In particular the probiotic is live probiotic bacteria non-replicating probiotic bacteria, dead probiotic bacteria, non-viable probiotic bacteria, and any combination thereof. More particularly the probiotic is live probiotic bacteria.

Preferably the probiotics comprise a combination of Lactobacillus and Bifidobacterium. The most preferred Lactobacillus strain is the Lactobacillus rhamnosus GG strain deposited by Nestlé R&D centre Shanghai Ltd (13 Qiao Nan, Cao An Road, Jiading District, Shanghai 201812, P.R. China) at the China General Microbiological Culture Collection Centre (CGMCC) and available under the deposit number CGMCC 1.3724. The most preferred Bifidobacterium strain is the Bifidobacterium lactis BB12 strain deposited at the Collection Nationale de Cultures De Microorganismes (CNCM) as CNCM I-3446. Preferably the probiotics comprise a mixture of the Lactobacillus rhamnosus GG strain available under the deposit number CGMCC 1.3724 and of the Bifidobacterium lactis BB12 strain deposited as CNCM I-3446. Most preferably the probiotics consist of a mixture of the Lactobacillus rhamnosus GG strain available under the deposit number CGMCC 1.3724 and of the Bifidobacterium lactis BB12 strain deposited as CNCM I-3446. In a preferred embodiment, the probiotic is provided in an amount of from $10^5$ to $10^{12}$ colony forming units (cfu) per daily dose, more preferably from $10^7$ to $10^{11}$ cfu per daily dose.

Additional vitamins and minerals may also be added. For example, vitamins and minerals may be added in accordance with the recommendations of Government bodies such as the USRDA. For example, the composition may contain one or more of the following micronutrients, calcium, magnesium, phosphorus, iron, zinc, copper, iodine, selenium, vitamin A or retinol activity equivalent (RAE) for example in the form of beta carotene or a mix of carotenoids, Vitamin C, Vitamin B1, niacin, folic acid, biotin, Vitamin E. Preferably the composition may contain one or more of the following micronutrients in the following amounts: 100 to 2500 mg calcium, 35 to 350 mg magnesium, 70 to 3500 mg phosphorus, 2.7 to 45 mg iron, 0.1 to 10 mg copper, 22 to 1,100 μg iodine, 6 to 400 μg selenium, 77 to 3000 μg of vitamin A or retinol activity equivalent (RAE) for example in the form of beta carotene or a mix of carotenoids, 8.5 to 850 mg Vitamin C, 0.14 to 14 mg Vitamin B1, 1.8 to 35 mg niacin, 60 to 1000 μg folic acid, 3 to 300 μg biotin, 1.9 to 109 μg Vitamin E.

The composition of the invention may advantageously further comprise at least one oil selected from long chain polyunsaturated fatty acids, such as arachidonic acid (ARA), eicosapentaenoic acid (EPA) and/or docosahexaenoic acid (DHA), in any suitable amount as known by the person skilled in the art for example in an amount of 100-500 mg per daily dose, more preferably between 200 and 400 mg per daily dose.

However, notwithstanding the above, a high fat intake and/or Maternal PUFA supplementation during pregnancy has been linked to an increased risk of higher adiposity in the offspring. Accordingly, in a particular embodiment the composition of the invention does not comprise a long chain polyunsaturated fatty acid and/or animal fats and/or vegetable fats.

In another particular embodiment the composition of the invention does not comprise an oligosaccharide.

Any of the nutrients mentioned herein may be used in any amount that is effective in achieving the objective of the present invention. Skilled artisans will be able to determine appropriate dosages. Typically, dosage will depend on age, size and health status of the mother, on her lifestyle, as well as on her genetic heritage.

Typically, amounts are defined in the present application as amounts per daily dose. The amount of nutrient may thus be selected in each composition accordingly depending upon whether it is intended to be consumed once a day or more or less frequently.

The nutrients may be provided as a sustained release formulation. This way, the nutrient can be consumed less frequently, while the body is still constantly supplied with sufficient amount of such nutrient.

The composition of the present invention is intended for maternal administration. This means that the composition is to be administered to a woman desiring to get pregnant, to a pregnant woman and/or to a lactating woman. Preferably, it is to be administered to a woman desiring to get pregnant and/or to a pregnant woman, most preferably to a pregnant woman.

Preferably, the composition of the invention is administered to a woman desiring to get pregnant, for example during at least 1, 2, 3 or 4 months preceding the pregnancy or desired pregnancy. When the composition is to be administered to a pregnant or lactating woman, the composition is preferably administered for at least 4, preferably at least 8, more preferably at least 12, more preferably at least 16, more preferably at least 20, more preferably at least 24, more preferably at least 28, even more preferably at least 36 weeks during pregnancy and or lactation. As the nutritional requirements increase in the second and third trimester of pregnancy, it is further preferred to administer the composition of the invention throughout the third trimester of pregnancy and most preferably throughout the second and third trimesters of pregnancy.

It is important that women desiring to get pregnant prepare their body for pregnancy by taking appropriate nutrients. Then, women's nutrient needs increase during pregnancy and lactation. Also lactation is demanding on maternal stores of energy, protein and other nutrients that need to be established and replenished. The composition of the present invention is specifically designed to meet the needs of the woman during each of these periods.

The composition can be in any form that is suitable to administer all the ingredients to the woman. For example it can be in the form of a powdered nutritional composition to be reconstituted in milk or water, a food product, a functional food product, a drink (beverage), a dairy product, a pharmaceutical formulation, a pet food product, a nutritional supplement or a nutraceutical.

When the composition is in the form of a powdered nutritional composition to be reconstituted in milk or water it preferably comprises a protein source, a carbohydrate source and a lipid source, preferably together with lecithin. More preferably it also comprises soya lecithin and/or a bulking agent. The protein source may be dried milk or dried skimmed milk. As carbohydrate source sucrose and/or maltodextrin may be used. The lipid source may be vegetable oil. The formulation may also alternatively or additionally contain glucose syrup, milk fat, magnesium citrate, choline salts and esters, prebiotic fibers, and/or ascorbyl palmitate. Flavor compounds, such as cocoa powder or honey, for example, may be added to provide taste variations.

The term "food product", as used herein, refers to any kind of product that may be safely consumed by a human or animal. Said food product may be in solid, semi-solid or liquid form and may comprise one or more nutrients, foods or nutritional supplements. For instance, the food product may additional comprise the following nutrients and micronutrients: a source of proteins, a source of lipids, a source of carbohydrates, vitamins and minerals. The composition may also contain anti-oxidants, stabilizers (when provided in solid form) or emulsifiers (when provided in liquid form).

The term "functional food product" as used herein, refers to a food product providing an additional health-promoting or disease-preventing function to the individual.

Food products and functional food products include for example cereal-based products, yogurts or other milk-derived products and bars.

The term "nutritional supplement", or "dietary supplement", as used herein, refers to a nutritional product that provides nutrients to an individual that may otherwise not be consumed in sufficient quantities by said individual. For instance, a nutritional supplement may include vitamins, minerals, fiber, fatty acids, or amino acids.

Supplements can for example be provided in the form of a pill, a tablet a lozenger, a chewy capsule or tablet, a tablet or capsule, or a powder supplement that can for example be dissolved in water or sprinkled on food. Most preferred is a powder supplement that can be dissolved in liquid or sprinkled on food, most preferably dissolved in water. Such supplements typically provide the selected nutrients while not representing a significant portion of the overall nutritional needs of the subject. Typically they do not represent more than 0.1%, 1%, 5%, 10% or 20% of the daily energy need of the subject.

The term "dairy products", as used herein, refers to food products produced from animals such as cows, goats, sheep, yaks, horses, camels, and other mammals. Examples of dairy products are low-fat milk (e.g. 0.1%, 0.5% or 1.5% fat), fat-free milk, milk powder, whole milk, whole milk products, butter, buttermilk, buttermilk products, skim milk, skim milk products, high milk-fat products, condensed milk, crème fraiche, cheese, ice cream and confectionery products, probiotic drinks or probiotic yoghurt type drinks.

The term "pharmaceutical formulation" as used herein, refers to a composition comprising at least one pharmaceutically active agent, chemical substance or drug. The pharmaceutical formulation may be in solid or liquid form and can comprise at least one additional active agent, carrier, vehicle, excipient, or auxiliary agent identifiable by a person skilled in the art. The pharmaceutical formulation can be in the form of a tablet, capsule, granules, powder, liquid or syrup. The term "beverage product" as used herein, refers to a nutritional product in liquid or semi-liquid form that may be safely consumed by an individual. The term "pet food product" as used herein refers to a nutritional product that is intended for consumption by pets. A pet, or companion animal, as referenced herein, is to be understood as an animal selected from dogs, cats, birds, fish, rodents such as mice, rats, and guinea pigs, rabbits, etc.

All ingredients of the composition can be admixed together or alternatively the composition can be provided in the form of a kit of parts wherein ingredients or groups of ingredients are provided separately and are intended to be consumed together by the woman.

Therefore a kit of parts comprising the ingredients of the composition of the present invention, and comprising at least two physically separated compositions each comprising at least one of the ingredients mentioned above, wherein at least one of the physically separated compositions comprises myo-inositol and at least one of the physically separated compositions comprises vitamin B2, is another object of the present invention.

Each of the physically separated compositions can further comprise one or more of the other ingredients mentioned above. Most preferably, a first composition comprises vitamin B2 and optionally other vitamins mentioned above and a second composition comprises probiotics. Myo-inositol can be provided in the first or the second composition or even separately, but is preferably provided in the same composition as the vitamins. In a preferred embodiment of the invention, the kit of parts comprises a first composition comprising myo-inositol and vitamin B2, optionally with any of the other vitamins and/or nutrients mentioned above, except probiotics, and a second composition comprising probiotics. In a most preferred embodiment, the kit of parts comprises a first composition comprising myo-inositol, vitamin B2, vitamin B6, vitamin B12, vitamin D and zinc and a second composition comprising probiotics. Separation of probiotics from other ingredients is mostly preferred to avoid any damage to the probiotics due to the presence of high concentration of minerals or other nutrients.

For the purpose of the present invention, the composition (or the compositions contained in the kit of part) is administered regularly, for example two times a day, daily, every two days or weekly.

In another embodiment, the present invention provides a process for the manufacture of a composition for maternal administration comprising admixing myo-inositol with vitamin B2 and optionally with any of the other ingredients mentioned above.

As stated above, in addition to the present inventors identifying that a composition for maternal administration comprising vitamin B2 and myo-inositol is of particular usefulness to address the specific nutritional needs of women desiring to get pregnant, of pregnant and lactating women and of their offspring, said inventors have also surprisingly found that the administration of vitamin B2 to a subject may enhance glucose uptake in mammalian cells. Even more surprisingly the inventors found that when vitamin B2 is used in combination with vitamin D or vitamin D and zinc, there may be an improved effect on the enhancement of glucose uptake in mammalian cells, in particular mammalian muscle cells. Accordingly, administering vitamin B2 to a subject before and/or during pregnancy may prevent and/or treat GDM and/or prevent a condition associated with GDM in a pregnant subject or its offspring.

As stated above, the inventors have surprisingly found that vitamin B2 may be used to enhance glucose uptake in mammalian cells, in particular in mammalian muscle cells. Accordingly, administering vitamin B2 to a subject before and/or during pregnancy may prevent and/or treat GDM and/or prevent a condition associated with GDM in a pregnant subject or its offspring. The inventors have also found that heterozygous db/db mice when administered a supplemental vitamin B2 during a preconception and a pregnancy period have a lower fasting glucose concentration in comparison to mice not administered a supplemental dose.

The term "subject" as used herein refers to a mammal and more particularly a cat, a dog or a human.

The term "GDM" as used herein refers to any degree of impaired glucose tolerance that onsets or is first recognized during pregnancy.

Whether or not a mammal has an impaired glucose tolerance may be determined by measuring its fasting glucose plasma concentration, or by carrying out an oral glucose tolerance test (OGTT). The skilled person will be familiar with these tests and the criteria for diagnosing an impaired glucose tolerance and hence GDM. According to the criteria set out in the National Academy of clinical biochemistry (NACB) guidelines and the international association of diabetes and pregnancy study group (IADPSG) guidelines, published by the American Association for clinical chemistry (AACC), a pregnant human subject is considered as having an impaired glucose tolerance if their fasting plasma glucose concentration equates to 5.1mmol/L or more, or if their blood glucose concentration is higher than 10 mmol/L 1hour after a 75gram glucose drink, or higher than 8.5 mmol/L 2 hours after a 75gram glucose drink.

The term "treat" as used herein encompasses amelioration and/or alleviation of a condition i.e. the amelioration and/or alleviation of the symptoms of a condition. It may for example encompass the reduction of the severity of a condition in a subject.

The term "prevent" as used herein refers to the prevention of the occurrence, or reduction of the risk of the occurrence, of a condition in a subject.

Any source or form of Vitamin B2 suitable for ingestion by the pregnant subject may be used. In particular vitamin B2 may be riboflavin e.g. riboflavin sold under the trademark Riboflavin Universal.

A metabolite of vitamin B2 can be selected from the group consisting of flavin mononucleotide (hereinafter FMN), Flavin Adenine Dinucleotide (hereinafter FAD), and salts thereof e.g. riboflavin-5'-phosphate sodium salt.

Any source or form of myo-inositol suitable for ingestion by the pregnant subject may be used in the invention.

A metabolite of myo-inositol can be selected from the group consisting of D-chiro-inositol, L-chiro-inositol and a combination thereof. In particular the metabolite is D-chiro-inositol.

A combination of vitamin B2 with myo-inositol (cis-1,2,3,5-trans-4,6-cyclohexanehexol) and/or one or more probiotic and/or one or more of vitamin B6, B12 and D and or zinc may be particularly effective for the treatment and/or prevention of GDM and conditions associated therewith in a pregnant subject and/or its offspring. The combination of vitamin B2 with these listed ingredients may provide an improved effect e.g. a more than additive effect or synergistic effect, over what could be expected on the basis of the effect of the individual ingredients when used alone to treat or prevent GDM and a condition associated therewith in a pregnant subject and/or its offspring.

Accordingly, the composition for maternal administration of the present invention comprises myo-inositol, vitamin B2, vitamin B12, vitamin D, Bifidobacterium lactis BB12 and Lactobacillus rhamnosus GG.

In particular the inventors have found that when vitamin B2 is used in combination with vitamin D there may be an improved enhancement in glucose uptake in comparison to when vitamin B2 or vitamin D is used alone. More particularly still, the inventors have found that when vitamin B2 is used in combination with vitamin D and zinc, there may be an improved enhancement in glucose uptake in comparison to when vitamin B2, zinc, or vitamin D is used alone, and that this improved enhancement may be more than additive.

Further, the inventors have found that mice administered myo-inositol in combination with supplemental vitamin B2 have a surprisingly low fasting glucose concentration during pregnancy and pre pregnancy, and further still, the inventors have found that heterozygous db/db mice when administered myo-inositol in combination with supplemental vitamin B2 during pregnancy have a surprisingly lower retroperitoneal fat deposition.

Any source or form of vitamins B6, B12 and D and or zinc suitable for ingestion by the pregnant subject may be used in the invention.

In particular, the vitamin B6 may be pyroxidine hydrochloride, and/or a metabolite of vitamin B6 selected from the group consisting of Pyridoxal 5'-phosphate (hereinafter PLP).

In particular, the vitamin B12 may be cyanocobalamin, and/or a metabolite of vitamin B12 selected from the group consisting of hydroxocobalamin, methylcobalamin, adenosylcobalamin, and a combination thereof.

In particular, the vitamin D may be vitamin D2, vitamin D3, a precursor of vitamin D selected from the group consisting of 7-dehydrocholecalciferol, a metabolite of vitamin D selected from the group consisting of 25-hydroxyvitamin D3, 1,25-dihydroxyvitamin D3, 25-hydroxyvitamin D2, 1,25-dihydroxyvitamin D2, and a combination of any of the foregoing

More particularly the vitamin D is vitamin D3, a metabolite of vitamin D selected from the group consisting of 25-hydroxyvitamin D3, 1,25-dihydroxyvitamin, and combination of any of the foregoing. Even more particularly the vitamin D3 is Cholecalciferol.

In particular the probiotic is live probiotic bacteria non-replicating probiotic bacteria, dead probiotic bacteria, non-viable probiotic bacteria, and any combination thereof. More particularly the probiotic is live probiotic bacteria.

The one or more probiotic bacteria may be any lactic acid bacteria, Bifidobacteria, or combination thereof, wherein said lactic acid bacteria and Bifidobacteria have established probiotic characteristics. Non limiting examples of lactic acid bacteria strains include; Lactobacillus rhamnosus ATCC 53103 obtainable inter alia from Valio Oy of Finland under the trade mark LGG and Lactobacillus rhamnosus deposited by Nestlé R&D centre Shanghai Ltd (13 Qiao Nan, Cao An Road, Jiading District, Shanghai 201812, P.R. China) at the China General Microbiological Culture Collection Centre (CGMCC) and available under the deposit number CGMCC 1.3724. None limiting examples of Bifidobacteria strains include; Bifidobacterium lactis deposited at the Collection Nationale de Cultures De Microorganismes (CNCM) CNCM I-3446 sold inter alia by the Christian Hansen company of Denmark under the trade mark Bb12, Bifidobacterium longum ATCC BAA-999 sold by Morinaga Milk Industry Co. Ltd. of Japan under the trade mark BB536, the strain of Bifidobacterium breve sold by Danisco under the trade mark Bb-03, the strain of Bifidobacterium breve sold by Morinaga under the trade mark M-16V and the strain of Bifidobacterium breve sold by Institut Rosell (Lallemand) under the trade mark R0070.

According to the present invention the one or more probiotic is a mixture of Lactobacillus rhamnosus CGMCC 1.3724 and Bifidobacterium lactis CNCM I-3446

The Lactobacillus rhamnosus CGMCC 1.3724 and Bifidobacterium lactis CNCM I-3446 can be used in a ratio of 1:99 to 99:1, however, more particularly they will be used in a ratio of 1:2 to 2 to 1, even more particularly 1:1.

The vitamin B2, in combination with myo-inositol, vitamin B6, vitamin B12, vitamin D, Bifidobacterium lactis BB12 and Lactobacillus rhamnosus GG can be employed in any effective dose that provides a benefit with respect to the treatment or prevention of GDM and a condition associated therewith in a pregnant subject and/or its offspring.

An effective dose may be any dose that improves, by any degree, an impaired glucose tolerance in a pregnant subject.

It is well within the purview of the skilled person to determine an effective dose. Typically, an effective dose will depend on the type, age, size and health status of the subject, on the subject's lifestyle, as well as on its genetic heritage.

An effective dose may, for example, be determined by testing the effect of a dose on a subject's fasting glucose plasma concentration, or by carrying out an OGTT. An effective dose should improve a subject's fasting glucose concentration and lower a subject's glycemic response.

A particularly useful dose of vitamin B2 may be 0.14 to 14 mg, 1 to 2.5mg, 1.5 to 2mg, or 1.8mg.

A particularly useful dose of myo-inositol, if present, is from 0.2 to 5mg, 1.5 to 5mg, 2 to 4g, or 2g.

A particularly useful dose of the one or more probiotic, if present, is from 10e5 to 10e12 colony forming units (cfu), or 10e7 to 10e11 cfu.

A particularly useful dose of vitamin B6, if present, is 0.19 to 19 mg, or 2.6mg. A particularly useful dose of vitamin B12, if present, is 0.26 to 26 $\mu$g, or 5.2 $\mu$g. A particularly useful dose of vitamin D, if present, is 1.5 to 100 $\mu$g, or 10 $\mu$g.

A particularly useful dose of zinc, if present, is 1.1 to 40 mg, or 10mg.

The term "dose" as used herein refers to a daily quantity that is administered to a subject before and/or during pregnancy and/or during lactation. The daily quantity or dose may be administered all at once or it may be spread out over several administrations throughout a day. The dose may be by administered by any known method, in particularly enterally e.g. orally.

The dose(s) may be administered at any time e.g. day time or night time. The dose may be particularly effective if administered before the subject has food or beverage e.g. has a meal or a snack.

In an embodiment the dose is spread over 2 administrations, in a particular the dose is spread over 2 administrations, one in the morning and one in the evening, in particularly before breakfast and before the evening meal.

The term "breakfast" as used herein refers to the first meal of the day.

The term "evening meal" as used herein refers the last meal of the day.

In another disclosure which is not part of the present invention there is provided a kit for for use to treat or prevent GDM and a condition associated therewith in a pregnant subject and/or its offspring, the kit comprising:

a. vitamin B2, and optionally myo-inositol and/or one or more probiotic, and/or one or more of vitamin B6, B12 and D and/or zinc

b. A label indicating dosage requirements for a pregnant subject.

The dosage requirements may be 2 administrations per day, in particular 2 administrations per day before any food or beverage. More particularly, 2 administrations per day wherein one is in the morning and one is in the evening. Even more particularly, 2 administrations per day wherein one is in the morning before breakfast and one is in the evening before the evening meal.

The vitamin B2, myo-inositol, one or more probiotic, and one or more of vitamin B6, B12 and D, may all be provided in a format providing sustained release of said vitamins or one or more probiotic. This way, these compounds can be consumed less frequently, while the body is still constantly supplied with sufficient amount them.

Vitamin B2 when used in combination with myo-inositol and/or one or more probiotic and/or one or more of vitamin B6, B12 and D and/ or zinc, may be administered in the same composition as said myo-inositol and/or one or more probiotic and/or one or more of vitamin B6, B12 and D and/or zinc. Alternatively, one or more of said myo-inositol and/or one or more probiotic and/or one or more of vitamin B6, B12 and D and/or zinc may be administered separately to said vitamin B2 e.g. vitamin B2 (optionally with myo-inositol, and one or more of vitamin B6, B12 and D and/or zinc) may be administered separately to said one or more probiotic. The separate administration may be simultaneously but separate administration e.g. vitamin B2 (optionally with myo-inositol, and one or more of vitamin B6, B12 and D and/or zinc) may be administered at the same time but separately to said one or more probiotic e.g. in two tablets administered separately and sequentially in quick succession e.g. within 5, 4, 3, 2, 1 minute, to said one or more probiotic.

The vitamin B2, optionally in combination with the myo-inositol and/or one or more probiotic, and/or one or more of vitamin B6, B12 and D and/or zinc, is intended for administration to a subject desiring to get pregnant, to a pregnant subject and/or to a lactating subject. In particular it is to be administered to a subject desiring to get pregnant and/or to a pregnant subject. More particularly it will be administered to a pregnant subject.

If vitamin B2, optionally in combination with the myo-inositol and/or one or more probiotic, and/or one or more of vitamin B6, B12 and D and/or zinc, is administered to a subject desiring to get pregnant it may for example be administered during at least 1, 2, 3 or 4 months preceding the pregnancy or desired pregnancy. If vitamin B2, optionally in combination with the myo-inositol and/or one or more probiotic, and/or one or more of vitamin B6, B12 and D and/or zinc is administered to a pregnant subject, it may be administered throughout or partially throughout the pregnancy e.g. for at least 4, at least 8, at least 12, at least 16, at least 20, at least 24, at least 28, or at least 36 weeks depending on the gestational period of the subject. Administration may also continue throughout or partially throughout the lactation period of said subject.

Since the risk of GDM increases in the second and third trimester of pregnancy, administration may be particularly beneficial in the second and third trimester of pregnancy.

The composition of the present invention can be employed in the form of a composition e.g. powdered nutritional composition, a food product, a functional food product, a drink (beverage), a dairy product, a pharmaceutical formulation, a pet food product, a nutraceutical, a nutritional supplement e.g. a powdered nutritional supplement e.g. to be sprinkled on food or dissolved in an aqueous medium e.g. water or juice, or milk.

When the composition is in the form of a powdered nutritional composition it may further comprises a protein source, a carbohydrate source and a lipid source, possibly together with lecithin. It may also comprise soya lecithin and/or a bulking agent. The protein source may be dried milk or dried skimmed milk. As carbohydrate source sucrose and/or maltodextrin may be used. The lipid source may be vegetable oil. The formulation may also alternatively or additionally contain glucose syrup, milk fat, magnesium citrate, choline salts and esters, prebiotic fibers, and/or ascorbyl palmitate. Flavor compounds, such as cocoa powder or honey, for example, may be added to provide taste variations.

The composition of the present invention may also be used in combination with other vitamins and minerals. For example, vitamins and minerals recommended by a governmental body, such as USRDA, for supplementation in pregnancy e.g. calcium, magnesium, phosphorus, iron, copper, iodine, selenium, vitamin A or retinol activity equivalent (RAE) e.g. beta carotene or a mix of carotenoids, Vitamin C, Vitamin B1, niacin, folic acid, biotin, Vitamin E.

It may be particularly beneficial if the composition of the invention is used in combination with one or more of the following micronutrients in the following amounts: 100 to 2500 mg calcium, 35 to 350 mg magnesium, 70 to 3500 mg phosphorus, 2.7 to 45 mg iron, 0.1 to 10 mg copper, 22 to 1,100 $\mu$g iodine, 6 to 400 $\mu$g selenium, 77 to 3000 $\mu$g of vitamin A or retinol activity equivalent (RAE) for example in the form of beta carotene or a mix of carotenoids, 8.5 to 850 mg vitamin C, 0.14 to 14 mg vitamin B1, 1.8 to 35 mg niacin, 60 to 1000 $\mu$g folic acid, 3 to 300 $\mu$g biotin, 1.9 to 109 $\mu$g Vitamin E.

The composition of the invention may also be advantageously used in combination with at least one oligosaccharide and/or at least one long chain polyunsaturated fatty acids, such as arachidonic acid (ARA), eicosapentaenoic acid (EPA) and/or docosahexaenoic acid (DHA). The oligosaccharides and long chain polyunsaturated fatty acids can be used in any concentration safe for administration to the subject.

The composition of the present invention may also be used in combination with other ingredients commonly used in the form of composition in which it is employed e.g powdered nutritional supplement, or a food product. Non limiting examples of such ingredients include: other nutrients, for instance, selected from the group of lipids (optionally in addition to DHA and ARA), carbohydrates, and protein, micronutrients (in addition to those set out above), or pharmaceutically active agents; conventional food additives such as anti-oxidants, stabilizers, emulsifiers, acidulants, thickeners, buffers or agents for pH adjustment, chelating agents, colorants, excipients, flavor agents, osmotic agents, pharmaceutically acceptable carriers, preservatives, sugars, sweeteners, texturizers, emulsifiers, water and any combination thereof.

However, as stated hereinabove, a high fat intake during pregnancy and/or Maternal PUFA supplementation has been linked to an increased risk of higher adiposity in the offspring. Accordingly, in a particular embodiment the composition of the invention is not used in combination with a long chain polyunsaturated fatty acid and/or animal fats and/or vegetable fats.

In a particular embodiment the composition of the invention is not used in combination with an oligosaccharide.

As stated hereinabove, GDM is associated with a variety of conditions that affect the pregnant subject and/or its offspring. GDM is considered as being associated with a condition if it increases the risk of a subject having or developing that condition during pregnancy, during birth, after birth or later in the life of said pregnant subject or its offspring.

Non limiting examples of conditions associated with GDM, that affect the pregnant subject and/or its offspring, include; preterm and caesarian delivery, birth injury to the mother or baby, shoulder dystocia, macrosomia, excessive offspring blood glucose concentration, excess weigh/adiposity and associated metabolic disorders e.g. type II diabetes, fatty liver disease and obesity, immediately after birth and later in the life of the offspring, and an increased risk for the mother of having or developing type 2 diabetes immediately after birth and later in life. Accordingly, by treating or preventing GDM, vitamin B2, optionally in combination with the myo-inositol and/or one or more probiotic, and or one or more of vitamin B6, B12 and D and/or zinc, may also be used to treat or prevent these conditions in these pregnant subjects or their offspring.

In another aspect not part of the present invention, the composition of the present invention is for use in the manufacture of a medicament for use in the treatment or prevention of GDM and a conditions associated therewith in a pregnant subject and/or its offspring, wherein said medicament is administered to said subject before and/or during pregnancy and/or during lactation.

GDM is more prevalent amongst subjects who suffered with it in a previous pregnancy, if the subject is overweight or obese, if there is a family history of GDM or type II diabetes. If the subject is human and over 25 years of age, and if the subject is human and is black, Hispanic, American Indian or Asian.

Accordingly, the composition of the invention may be particularly beneficial for treating and/or preventing GDM in one or more of these subsets of subjects.

Those skilled in the art will understand that they can freely combine all features of the present invention disclosed herein. In particular, features described for different embodiments of the present invention may be combined. Where known equivalents exist to specific features, such equivalents are incorporated as if specifically referred to in this specification. Further advantages and features of the present invention are apparent from the figure and non-limiting example.

The present invention will now be described in further details by the way of the following examples.

## Examples

### Example 1

An example composition comprising vitamin B2 in combination with myo-inositol, vitamins B6, B12 and D, and Lactobacillus rhamnosus GG[1] and Bifidobacterium lactis BB12[2] is set out in table 1.

The composition in table I is for a nutritional supplement in a powder form, intended to be sprinkled on food.

Table 1 : Composition of Example 1

| Ingredient | Amount per daily dose |
|---|---|
| Myo-inositol | 4g |
| Vitamin D | 10 µg |
| Vitamin B6 | 2.6 mg |
| Vitamin B12 | 5.2 µg |
| Vitamin B2 | 1.8 mg |
| Zinc | 10 mg |
| β-carotene | 720 µg |
| Folic acid | 400 µg |
| Iron | 12 µg |
| Calcium | 150 µg |
| Iodine | 150 µg |
| Lactobacillus rhamnosus GG[1] | $1 \times 10^9$ cfu |
| Bifidobacterium lactis BB12[2] | $1 \times 10^9$ cfu |
| 1) Strain deposited as CGMCC 1.3724<br>2) Strain deposited as CNCM I-3446 | |

The composition may be provided as a kit of parts comprising in one sachet the probiotic as a powder and in a second sachet all other ingredients.

### Example 2

A milk powder drink for pregnant women to be reconstituted in water is provided consisting of 30g of milk powder per serving admixed with the ingredients listed in Table 1, in half of the amounts mentioned in Table 1. The composition is to be administered to a pregnant woman twice per day during at least the third trimester of pregnancy.

### Example 3

The effect of vitamin B2 on glucose uptake in L6 differentiated muscle cells was measured after short (2 hrs) and long term (24 hrs) incubations.

Ingredient G refers to vitamin B2, ingredient k refers to 1,25-dihydroxyvitamin D3, and ingredient J refers to zinc chloride.

Ingredient G was tested at 1 µM. Ingredient K was tested at 10 nM, and ingredient J was tested at 20 µM.

The day of the experiment, the ingredients were diluted in a serum-free medium (DMEM) containing 5.5 mM D-glucose to achieve the final tested concentrations and 0.1 % of DMSO.

L6 myoblasts were maintained in growth medium or DMEM 25 mM glucose supplemented with 10 % of fetal bovine serum (FBS), 100 U/ml penicillin, and 100 µg/ml streptomycin. For the glucose uptake study, cells were grown on 24 well plates at a density of 1000-1500 cells/well in 0.5 mL of growth medium. Five days after plating, the differentiation induction into myotubes was carried out in DMEM 25 mM glucose containing 2 % FBS, 100 U/ml penicillin, and 100

μg/ml streptomycin at 37°C under a 5 % CO₂ atmosphere. The culture medium was changed every other day.

For short term incubation (2 hrs), the day prior to the test, the culture medium was replaced by DMEM 25 mM D-glucose containing 0.25 % BSA, 100 U/ml penicillin, and 100 μg/ml streptomycin. The day after and prior to the test, cells were washed and incubated in DMEM containing 5.5 mM D-glucose, 0.25 % BSA in the presence of the ingredients for 2 hours without insulin.

For long term incubation (24 hrs), the day prior to the test, cells were pre-incubated in the presence of the tested ingredients (ingredient J, K, or a combination thereof) for 22 hours in DMEM 25 mM D-glucose containing 0.25 % BSA, 100 U/ml penicillin, and 100 μg/ml streptomycin in the absence of insulin. The day after and prior to the test, cells were washed and incubated in the presence of the tested ingredients for 2 hours more in DMEM containing 5.5 mM D-glucose, 0.25 % BSA without insulin.

The glucose uptake was measured by incubation of the cells with radiolabelled 2-deoxy-D-[1,2 3H] glucose for 15 min. Glucose uptake was arrested by two washing steps in ice-cold PBS 1X. Then cells were solubilized in 0.1 N NaOH for 30 min. Cell associated radioactivity was then counted and protein quantification was determined using the coloric Lowry method.

Each ingredient was tested in triplicate.

Results are expressed in pmol glucose incorporated / min / mg protein and in % of control or basal condition (100 %).

A statistical analysis allowing multiple comparisons (one way ANOVA followed by a Dunnett's t test; $*P < 0.05$, $**P < 0.01$, $***P < 0.001$ versus control condition) was carried out. All ingredients showing significant increase in glucose uptake compared to control or basal condition are considered active.

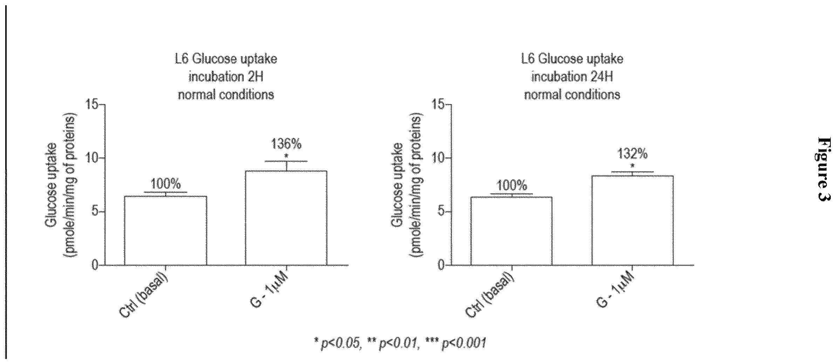

The results are shown in Figures 3 to 5.

As can be seen from figure 3, increases in glucose uptake were observed in L6 differentiated muscle cells with ingredient G after short term incubation (2 hrs) and long term incubation (24 hrs) in the absence of insulin.

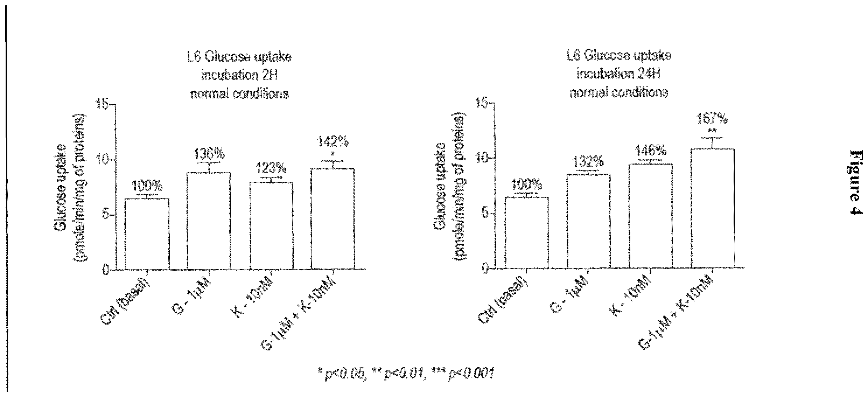

As can be seen from figure 4, increases in glucose uptake were observed in L6 differentiated muscle cells with the combination of ingredient G and ingredient K after short term incubation (2 hrs) and long term incubation (24 hrs) in the absence of insulin.

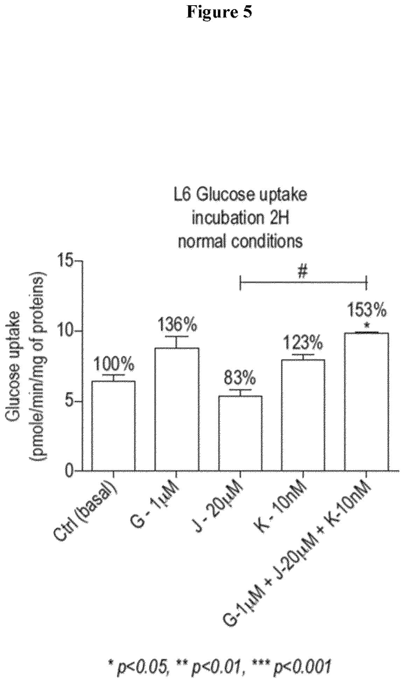

As can be seen from figure 5 increases in glucose uptake were observed in L6 differentiated muscle cell with the combination of ingredient G, K and ingredient J after short term incubation (2 hrs) in the absence of insulin.

## Example 4

### Animals

Male and female heterozygous db/db mice (Leprdb/+) were originally sourced from Jackson Laboratories (USA), and a breeding colony established (Ruakura, New Zealand).

Female mice were genotyped and then weaned at 21 days of age, and both WT (Lepr+/+) and heterozygous (Leprdb/+) mice were placed on AIN-93G diet (composition specified in table 2). At 28 days of age an oral glucose tolerance test (OGTT) was performed, then mice were randomized to one of 4 diets

    1 - AIN-93G control
    2 - AIN-93G supplemented with myo-inositol
    3 - AIN-93G supplemented with Vitamin B2
    4 - AIN-93G supplemented with myo-inositol and Vitamin B2

The composition of AIN-93G is specified in table 2.

A further OGTT was performed before and following the experimental diet).

All OGTT's were performed in the same way. Briefly, mice were fasted for 6 hours (from 7am to 1pm). Fasting glucose was measured from blood taken the end of the tail using a hand-held glucometer (Freestyle Optimum glucometer by Abbott). The mouse then received a glucose challenge - 2g/kg delivered by gavage. The blood glucose concentration measured using the glucometer, at 30, 60, 90 and 120 minutes following the glucose challenge.

At 12 weeks of age, females were housed with a genotype-matched male until a vaginal plug was observed, indicating day 0.5 of gestation (term pregnancy = 19.5 days). Female mice remained on their allocated experimental diet throughout gestation. An OGTT was performed at gestational day 16.5.

At gestational day 18.5, mice were culled after a 6 hour fast. Fasting blood samples and retroperitoneal fat pad were harvested.

Table 2 : AIN-93G Diet

| Product # D10012G | | gm% | Kcal% |
|---|---|---|---|
| Protein | | 20 | 20.3 |
| Carbohydrate | | 64 | 63.9 |
| Fat | | 7 | 15.8 |
| | Total kcal/gm | 3.9 | |
| Ingredient | | gm | Kcal |
| Casein, 30 Mesh | | 200 | 800 |
| L-Cystine | | 3 | 12 |
| Corn Starch | | 397 | 1590 |
| Maltodextrin | | 132 | 528 |
| Sucrose | | 100 | 400 |
| Cellulose | | 50 | 0 |
| Soybean Oil | | 70 | 630 |
| t-Butylhydroquinone | | 0.014 | 0 |
| Mineral mix S10022G | | 35 | 0 |
| Vitamin mix V10037 | | 10 | 40 |
| Choline Bitartrate | | 2.5 | 0 |
| Total | | 1000 | 4000 |

Table 2b - Vitamin mix added to AIN-93G

| Ingredient | gm | Amount in 10gm |
|---|---|---|
| Vitamin A, Acetate (500,000 IU/gm) | 0.8 | 4000 IU |
| Vitamin D3 (100,000 IU/gm) | 1 | 1,000 IU |
| Vitamin E Acetate (500 IU/gm) | 15 | 75 IU |
| Phylloquinone | 0.075 | 0.75 mg |
| Biotin, 1.0% | 2 | 0.2 mg |
| Cyanocobalamin, 0.1% | 2.5 | 25 $\mu$g |
| Folic Acid | 0.2 | 2 mg |
| Nicotinic Acid | 3 | 30 mg |
| Calcium Pantothenate | 1.6 | 16 mg |
| Pyridoxine---HCl | 0.7 | 7 mg |
| Riboflavin | 0.6 | 6 mg |
| Thiamin HCl | 0.6 | 6 mg |
| Sucrose | 971.925 | |
| TOTAL | 1000 | |

Table 2c-Supplemented amounts of myo-inositol and Vitamin B2 in experimental diets

| Diet Group | Amount of Myo-inositol present | Amount of Vitamin B2 present |
|---|---|---|
| 1 | 0g | 6mg / kg diet |
| 2 | 10g / kg diet | 6mg / kg diet |
| 3 | 0g | 24mg / kg diet |
| 4 | 10g / kg diet | 24mg / kg diet |

**Results**

Table 3 - blood glucose concentration and AUC for WT and heterozygous mice at 4 weeks of age. Mean +/- s.e.m.

| Genotype | Blood glucose at 0 mins (mmol/L) | Blood glucose at 30 mins (mmol/L) | Blood glucose at 60 mins (mmol/L) | Blood glucose at 90 mins (mmol/L) | Blood glucose at 120 mins (mmol/L) | AUC |
|---|---|---|---|---|---|---|
| WT | 11.6+/-0.7 | 17.5 +/- 0.8 | 14.0 +/- 0.6 | 12.6 +/- 0.5 | 12.0 +/- 0.5 | 1710 +/- 59 |
| Het | 11.0+/-0.5 | 17.7 +/- 0.9 | 14.2 +/- 0.7 | 13.1 +/-0.7 | 13.1 +/-0.6 | 1710 +/- 68 |

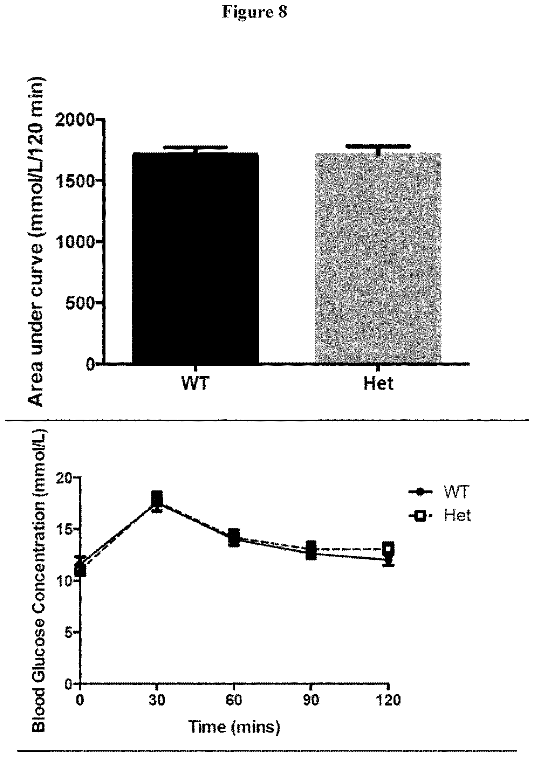

As can be seen from table 3 and figure 8, prior to randomisation to experimental diets, there were no differences in the OGTT between WT and heterozygous females.

Table 4 - fasting blood glucose (gestational day 18.5) measurements - data presented as mean +/- s.e.m.

| Diet group | Fasting blood glucose at day 18.5 (mmol/L) |
|---|---|
| WT group 1 | 7.1 +/- 0.9 |
| Het group 1 | 9.2 +/- 0.7 |
| Het group 2 | 8.2 +/- 0.8 |
| Het group 3 | 8.1 +/- 0.8 |
| Het group 4 | 6.9 +/- 1.0 |

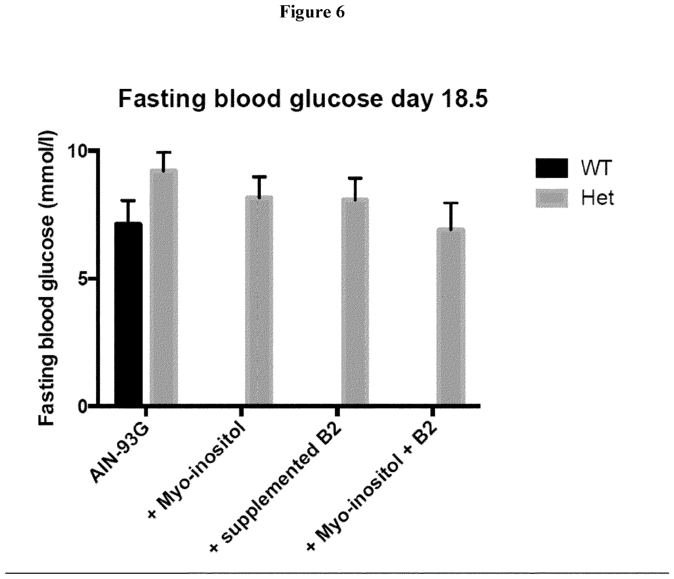

As can be seen in figure 6 and from the data in table 4, the Het mice administered a combination of vitamin B2 and myo-inositol appeared to have a lower fasting blood glucose level at the end of gestation in comparison to Het mice administered myo-inositol or probiotics alone, or the Het mice not administered myo-inositol or B2.

Table 5 - weight of retroperitoneal fat pads and gonadal fat, mean +/- s.e.m.

| Diet group | Weight of retroperitoneal fat pads (g) | Gonadal fat (g) |
|---|---|---|
| WT group 1 | 0.078 +/- 0.008 | 0.24 +/- 0.02 |
| Het group 1 | 0.152 +/- 0.041 | 0.32 +/- 0.04 |
| Het group 2 | 0.120 +/- 0.021 | 0.30 +/- 0.04 |
| Het group 3 | 0.164 +/- 0.040 | 0.42 +/- 0.05 |
| Het group 4 | 0.112 +/- 0.010 | 0.27 +/- 0.04 |

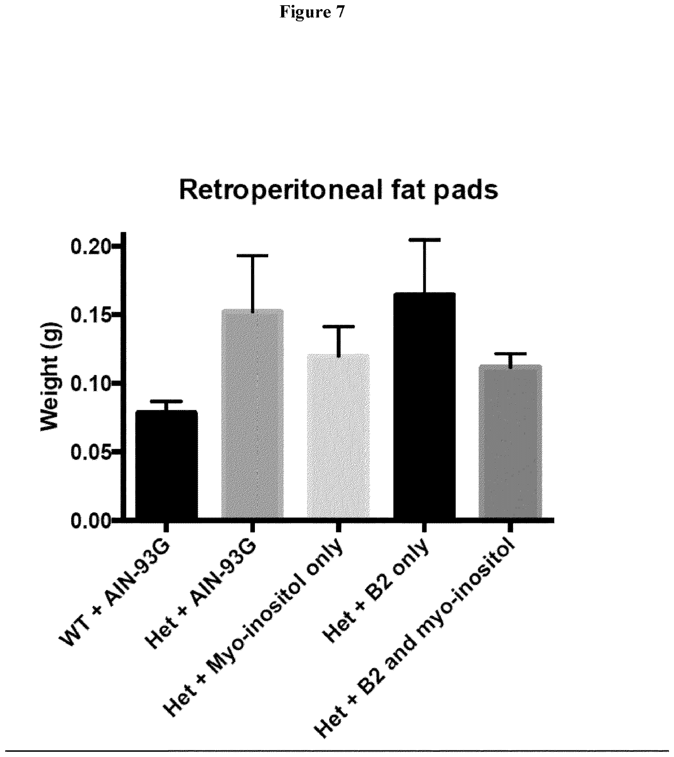

As can be seen in figure 7 and/or from the data in table 5, the Het mice administered a combination of vitamin B2 and myo-inositol appeared to have a lower retroperitoneal fat pads weight and a lower gonadal fat weight in comparison to Het mice administered myo-inositol or probiotics alone, or the Het mice not administered myo-inositol or B2.

Table 6 - Fasting blood glucose concentration following OGTT at 12 weeks of age. Mean +/- s.e.m.

| Diet group | 0 mins (mmol/L) | 30 mins (mmol/L) |
|---|---|---|
| WT group 1 | 9.1 +/- 1.1 | 21.7 +/-1.9 |
| Het group 1 | 8.3 +/- 0.4 | 17.6 +/-1.2 |
| Het group 2 | 7.5 +/- 0.8 | 16.7 +/-1.2 |
| Het group 3 | 8.4 +/- 1.0 | 17.1 +/-1.5 |
| Het group 4 | 6.9 +/- 0.8 | 16.6 +/- 1.9 |

As can be seen from the data in table 6, the Het mice administered vitamin B2 and/ or a combination of vitamin B2 and myo-inositol appeared to have a lower fasting blood glucose concentration at 12 weeks of age (prior to gestation) in comparison to Het mice not administered myo-inositol or B2, or Het mice administered myo-inositol .

Table 7 - Plasma leptin concentrations, measured at gestational day 18.5 (Mean +/- s.e.m)

|  | Leptin (ng/ml) |
| --- | --- |
| WT group 1 | 141.8 +/- 18.6 |
| Het group 1 | 212.6 +/- 22.4 |
| Het group 2 | 242.4 +/- 0.0 |
| Het group 3 | 217.5 +/- 13.0 |
| Het group 4 | 118.2 +/- 21.3 |

As can be seen from the data in table 7 and figure 17, the Het mice administered vitamin B2 and myo-inositol appeared to have lower plasma leptin concentrations at day 18.5 of gestation in comparison to Het mice not administered myo-inositol or B2, or Het mice administered myo-inositol .

Table 8 - Oral glucose tolerance test results - measured at gestational day 16.5 (mean +/- s.e.m)

|  | Blood glucose 0 mins (mmol/L) | Blood glucose 30 mins (mmol/L) | Blood glucose 60 mins (mmol/L) | Blood glucose 90 mins (mmol/L) | Blood glucose 120 mins (mmol/L) | AUC |
| --- | --- | --- | --- | --- | --- | --- |
| WT group 1 | 7.6 +/- 0.5 | 17.3 +/-1.3 | 11.9+/-0.9 | 10.4 +/- 0.7 | 10.4 +/- 0.7 | 1438 +/- 86 |
| Het group 1 | 7.2 +/- 0.4 | 17.3 +/-1.4 | 12.9 +/- 0.6 | 11.2 +/- 0.7 | 11.2 +/- 0.7 | 1506 +/- 81 |
| Het group 2 | 7.1 +/- 0.8 | 16.0 +/- 2.7 | 13.1 +/- 2.6 | 10.2 +/- 1.3 | 10.2 +/- 1.3 | 1439 +/- 158 |
| Het group 3 | 7.0 +/- 0.5 | 13.8 +/- 1.7 | 11.5 +/- 0.5 | 10.1 +/- 0.4 | 10.1 +/- 0.4 | 1340 +/- 65 |
| Het group 4 | 7.2 +/- 0.7 | 15.7 +/- 1.2 | 11.6 +/- 1 | 10.4 +/- 0.6 | 10.4 +/- 0.6 | 1376 +/- 86 |

As can be seen from the data in table 8, the Het mice administered B2 and/ or a combination of vitamin B2 and myo-inositol appeared to have lower fasting blood glucose concentrations and AUC at day 16.5 of gestation in comparison to Het mice not administered myo-inositol or B2.

**Example 5**

The effect of vitamin B2 on glucose uptake in insulin resistant L6 differentiated muscle cells after short (2 hrs) and long term (24 hrs) incubation in the absence of insulin was measured.

Ingredient G refers to vitamin B2 and it was tested at 1 $\mu$M.

The day of the experiment, ingredient G was diluted in a serum-free medium (DMEM) containing 5.5 mM D-glucose to achieve the final tested concentration and 0.1 % of DMSO.

L6 myoblasts were maintained in growth medium or DMEM 25 mM glucose supplemented with 10 % of fetal bovine serum (FBS), 100 U/ml penicillin, and 100 $\mu$g/ml streptomycin. For the glucose uptake study, cells were grown on 24 well plates at a density of 1000-1500 cells/well in 0,5mL of growth medium. Five days after plating, the differentiation induction into myotubes was carried out in DMEM 25 mM glucose containing 2 % FBS, 100 U/ml penicillin, and 100 $\mu$g/ml streptomycin at 37°C under a 5 % CO2 atmosphere. The culture medium was changed every other day.

For short term incubation (2 hours), the day prior to the test, cells were cultured in DMEM 25 mM D-glucose containing 0.25 % BSA, 100 U/ml penicillin, and 100 $\mu$g/ml streptomycin for 18 hours. The day after and prior to the test, cells were washed and incubated for 6 hours more in DMEM containing 5.5 mM D-glucose, 0.34 % BSA, 300$\mu$M palmitate (PALM) without insulin. 2 hours before the end of the incubation time, vitamin B2 was added to the media at a final concentration of 1 $\mu$M.

For long term incubation (24 hours), the day prior to the test, cells were pre-incubated in the presence of vitamin

B2 (1 μM) for 18 hours in DMEM 25 mM D-glucose containing 0.25 % BSA, 100 U/ml penicillin, and 100 μg/ml streptomycin in the absence of insulin. The day after and prior to the test, cells were washed and incubated in the presence of vitamin B2 (1 μM) for 6 hours more in DMEM containing 5.5 mM D-glucose, 0.34 % BSA, 300μM palmitate (PALM) without insulin.

Glucose uptake was measured by incubation of the cells with radiolabelled 2-deoxy-D-[1,2 3H] glucose for 15 min. Glucose uptake was arrested by two washing steps in ice-cold PBS 1X. Then cells were solubilised in 0.1 N NaOH for 30 min. Cell associated radioactivity was then counted and protein quantification was determined using the coloric Lowry method.

Each ingredient was tested in triplicate.

Results are expressed in pmol glucose incorporated / min / mg protein and in % of control.

A statistical analysis allowing multiple comparisons (one way ANOVA followed by a Dunnett's t test; $*P < 0.05$, $**P < 0.01$, $***P < 0.001$ versus control condition) was carried out. An ingredient showing a significant increase in glucose uptake compared to a control is considered active.

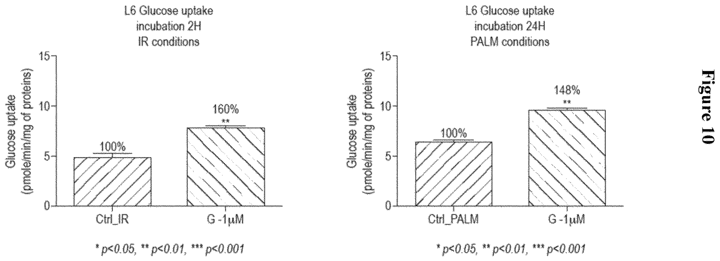

The results are shown in Figure 10.

**Example 6**

80 Females virgin Goto Kakizaki (GK) of 4-5 weeks old with close body weight, were purchased from Charles River (France and USA), caged singly in a temperature-controlled room (22±1°C) with a 12-h light/dark cycle, and maintained on a AIN-93G growing rodent diet (Research diets, USA, **Table 9**).

After one week on acclimation, GK rats were divided into 4 groups (n=20) after a randomization based on AUC glucose value and body weight, and received their corresponding treatment for 10 weeks as below :

1. Group Control: GK female rats ate *ad-libitum* a AIN-93G diet + a control gelatin **(Table10)**.
2. Group Myo-inositol : GK female rats ate *ad-libitum* a AIN93-G diet supplemented with myo-inositol diet (1 g myo-inositol* for 100 g de diet) + a control gelatin **(Table10)**.
3. Group Probiotic : GK female rats ate *ad-libitum* a AIN93-G + a gelatin containing $10^9$ LPR CFU and $10^9$ B.lactis CFU**(Table10)**.
4. Group Mix : GK female ate *ad-libitum* a AIN93G diet supplemented with myo-inositol diet (1 g myo-inositol for 100g de diet) + a gelatin containing $10^9$ LPR CFU and $10^9$ B.lactis CFU **(Table10)**.

*Kirsh Pharma

At 15 weeks of age, females were housed with Wistar males (Charles Rivers, France) until a vaginal plug was observed, indicating day 0.5 of gestation. Female rats remained on their allocated experimental diet throughout gestation. At 16.5 days of gestation an OGTT was performed on all females (n=20 per group) and at 19.5 days of gestation half of the animals (n=9-11 per group) were sacrificed after 6 hours fasting.

Table 9 : AIN-93G Research Diet

| Product # D10012G | | gm% | Kcal% |
|---|---|---|---|
| Protein | | 20 | 20.3 |
| Carbohydrate | | 64 | 63.9 |
| Fat | | 7 | 15.8 |
| | Total kcal/gm | 3.9 | |
| Ingredient | | gm | Kcal |

| Casein, 30 Mesh | | 200 | 800 |
|---|---|---|---|
| L-Cystine | | 3 | 12 |
| Corn Starch | | 397 | 1590 |
| Maltodextrin | | 132 | 528 |
| Sucrose | | 100 | 400 |
| Cellulose | | 50 | 0 |
| Soybean Oil | | 70 | 630 |
| t-Butylhydroquinone | | 0.014 | 0 |
| Mineral mix S10022G | | 35 | 0 |
| Vitamin mix V10037 | | 10 | 40 |
| Choline Bitartrate | | 2.5 | 0 |
| Total | | 1000 | 4000 |

Table 10 : Composition of the experimental Gelatin

| | Treatment | Control |
|---|---|---|
| Gelatin (g) | 12 | 12 |
| Sacharine (mg) | 0.06 | 0.06 |
| Probiotic (g)* | 7.7 | 0 |
| Maltodextrin mix (g) | 0 | 6.7 |
| TS + Water (ml) | 80.2 | 81.2 |
| Total (ml) | 99.96 | 99.96 |
| *Premix containing 2.53 E+10 cfu/g Lactobacillts rhamnosus (NCC4007) and 1.58E+10 cfu/g of Bifido Lactis (NCC2818). | | |

Measurements and analysis

Physiological measurements and sample collections:

- An oral glucose tolerance test (OGTT) was performed after 6 hours of day deprivation (from 7.30 am to 13.30 pm) in all animals at age of 5 weeks and at 16.5 days of gestation. During OGTT two baseline blood samples were taken from the tail vein, with at least 10 minutes interval between sampling (time -10 and 0), followed by an oral gavage of glucose solution (20% wt/v) at dose of 1.5 g/Kg body weight. Then further blood samples were collected from tail incision at 15, 30, 45, 60, 90 and 120 min after glucose administration. All blood samples were analysed directly for blood glucose level, using Contour Next glucometers (Bayer AG, Zurich, Switzerland).

- The body weight and food intake of the animals were measured 2 times / week throughout the study.

- Body composition (body fat, lean mass and body water content) was measured before and after 10 weeks of treatment by using nuclear magnetic resonance (EchoMR! TM 2004, Echo Medical Systems, Houston, USA). From these data collected it was possible to calculate the delta %fat and the fat gain / weight gain ratio as following :

- 

The delta %fat: (% fat at the end of the treatment - % fat before the start of the treatment)

- Fat gain / weight gain : ( fat (g) at the end of the treatment period - fat(g) before the start of the treatment) / (body weight (g) at the end of the treatment period - body weight (g) before the start of the treatment)

- lean gain / weight gain : (lean (g) at the end of the treatment period - lean (g) before the start of the treatment) / (body weight (g) at the end of the treatment period - body weight (g) before the start of the treatment)

- Fat gain : fat (g) at the end of the treatment period - fat(g) before the start of the treatment

- lean gain : lean (g) at the end of the treatment period - lean (g) before the start of the treatment

- % fat mass : fat mass (g) / body weight*100

- % lean mass : lean mass (g) / body weight*100

Sacrifice: dams were decapitated after 6 hours of day deprivation (from 7.30 am to 13.30 pm) and organ collected by dissection, weighted and immediately frozen in liquid nitrogen. They were kept at -80°C until analysis.

- Insulin pancreatic measurement: . Pancreas was weighted during the sacrifice and preserved in an acid-ethanol-H2O solution until insulin extraction For determination of pancreatic insulin, pancreas was homogenized with an acid-ethanol (solution v/v: 75% ethanol, 1.5% of 37% HCl and 23.5% distilled water) and incubated at -20°C overnight. The insulin content in the supernatant was measured by an ELISA method using kits from Crystal Chem. Inc (IL, USA).

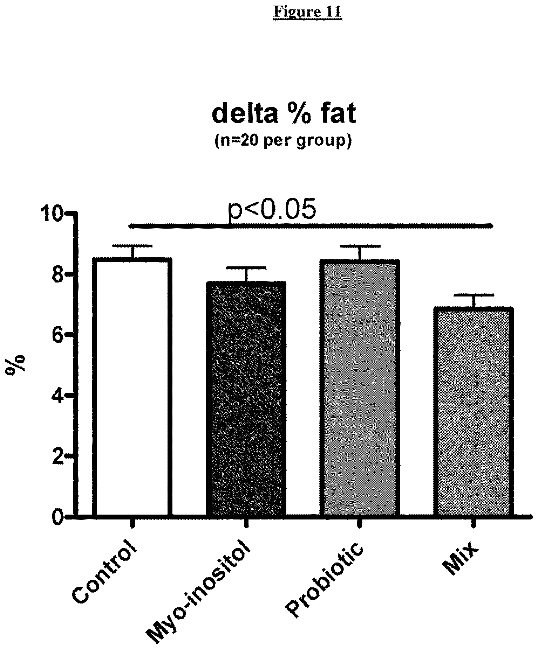

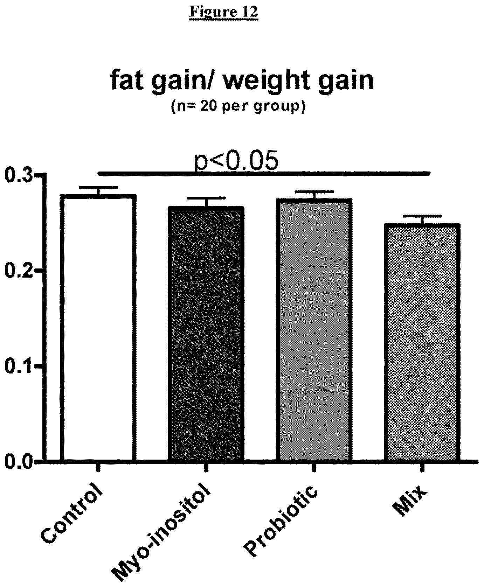

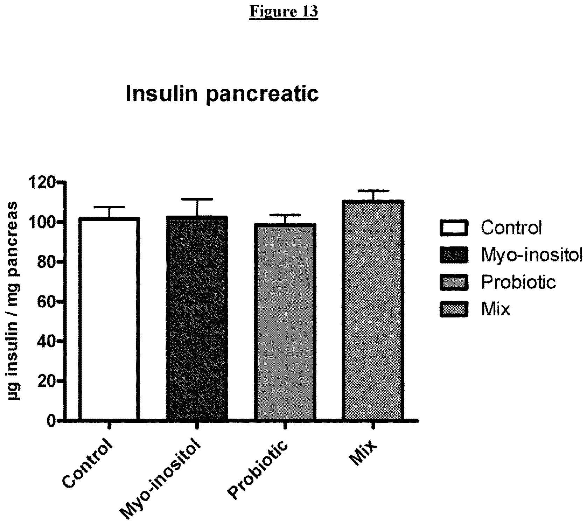

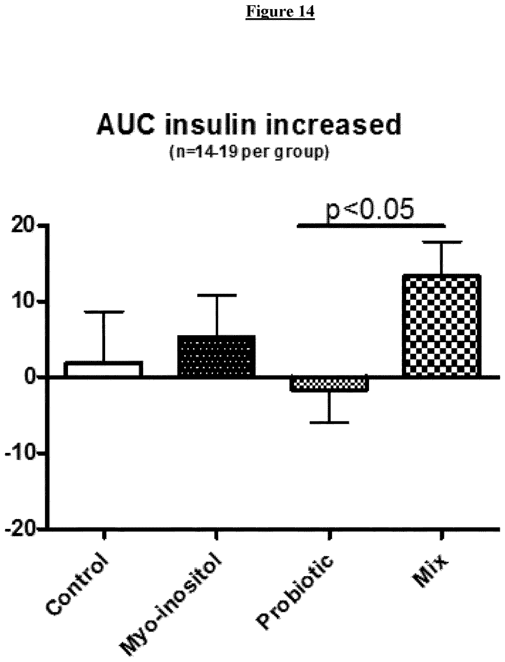

Results: The mean value for each measurement is shown in table 9. Results for delta % fat, fat gain/ weight gain, $\mu$g insulin pancreas/ g pancreas can also be seen in Figures 11 to 14.

**Table 11**

|  | Control | Myo-inositol | Probiotics | Mix (myo-inositol+probiotics) |
|---|---|---|---|---|
| % fat mass | 19.5 | 18.7 | 19.3 | 18.1 |
| % lean mass | 66.4 | 67.8 | 67.3 | 68.3 |
| Delta % fat | 8.8 | 7.7 | 8.4 | 6.8 |
| Fat gain/ weight gain | 0.28 | 0.27 | 0.27 | 0.24 |
| Lean gain/ weight gain | 0.57 | 0.58 | 0.58 | 0.60 |
| % fat gain | 27.6 | 26.6 | 27.3 | 24.7 |
| % lean gain | 57.4 | 58.3 | 58.5 | 60.3 |
| Body weight gain | 107 | 100 | 105 | 102 |

(continued)

|  | Control | Myo-inositol | Probiotics | Mix (myo-inositol+probiotics) |
|---|---|---|---|---|
| Fat gain (g) | 29.8 | 26.8 | 28.8 | 25.3 |
| Lean gain (g) | 61.7 | 58.5 | 61.3 | 61.5 |
| $\mu$g insulin pancreas/ g pancreas | 101 | 101 | 98.5 | 110.3 |
| AUC insulin increase | 2 | 5 | -2 | 13 |
| FGIR | 3.44 | 3.42 | 3.24 | 4.21 |
| HOMA1-IR | 10.42 | 9.29 | 9.76 | 9.07 |

**Claims**

1. A composition for maternal administration comprising myo-inositol and vitamin B2 and comprising vitamin B6, vitamin B12, vitamin D, and a combination of Lactobacillus and Bifidobacterium, wherein the Bifidobacterium is the Bifidobacterium lactis BB12 strain deposited as CNCM I-3446 and the Lactobacillus is the Lactobacillus rhamnosus GG strain available under the deposit number CGMCC 1.3724.

2. A composition according to claim 1, **characterized in that** the composition is to be administered to a woman desiring to get pregnant, to a pregnant woman or to a lactating woman.

3. A composition according to any of the preceding claims wherein said composition is in the form of a powdered nutritional composition to be reconstituted in milk or water, a food product, a drink, a nutritional supplement or a nutraceutical.

4. A kit of parts comprising:

   a) the ingredients myo-inositol, vitamin B2, vitamin B6, vitamin B12, vitamin D, and a combination of Lactobacillus and Bifidobacterium, wherein the Bifidobacterium is the Bifidobacterium lactis BB12 strain deposited as CNCM I-3446 and the Lactobacillus is the Lactobacillus rhamnosus GG strain available under the deposit number CGMCC 1.3724; and
   b) at least two physically separated compositions, each comprising at least one of said ingredients, **characterized in that** at least one of the physically separated compositions comprises myo-inositol and at least one of the physically separated compositions comprises vitamin B2.

**Patentansprüche**

1. Zusammensetzung für eine Verabreichung an die Mutter, umfassend Myo-Inositol und Vitamin B2 und umfassend Vitamin B6, Vitamin B12, Vitamin D und eine Kombination aus einer Laktobakterie und einem Bifidobakterium, wobei es sich bei dem Bifidobakterium um den Stamm Bifidobacterium lactis BB12 handelt, der als CNCM I-3446 hinterlegt ist, und es sich bei der Laktobakterie um den Stamm Lactobacillus rhamnosus GG handelt, der unter der Hinterlegungsnummer CGMCC 1.3724 verfügbar ist.

2. Zusammensetzung nach Anspruch 1, **dadurch gekennzeichnet, dass** die Zusammensetzung einer Frau, die wünscht, schwanger zu werden, einer schwangeren Frau oder einer stillenden Frau zu verabreichen ist.

3. Zusammensetzung nach einem der vorstehenden Ansprüche, wobei die Zusammensetzung in der Form einer pulverförmigen Nährstoffzusammensetzung, die in Milch oder Wasser zu rekonstituieren ist, eines Nahrungsmittelprodukts, eines Getränks, eines Nahrungsergänzungsmittels oder eines funktionellen Lebensmittels vorliegt.

4. Kit aus Teilen, umfassend:

   a) die Bestandteile Myo-Inositol, Vitamin B2, Vitamin B6, Vitamin B12, Vitamin D und eine Kombination aus der Laktobakterie und dem Bifidobakterium, wobei es sich bei dem Bifidobakterium um den Stamm Bifidobacterium lactis BB12 handelt, der als CNCM I-3446 hinterlegt ist, und es sich bei der Laktobakterie um den Stamm Lactobacillus rhamnosus GG handelt, der unter der Hinterlegungsnummer CGMCC 1.3724 verfügbar ist; und

b) mindestens zwei physisch getrennte Zusammensetzungen, jeweils umfassend mindestens einen der Bestandteile, **dadurch gekennzeichnet, dass** mindestens eine der physisch getrennten Zusammensetzungen Myo-Inosit umfasst und mindestens eine der physisch getrennten Zusammensetzungen Vitamin B2 umfasst.

**Revendications**

1. Composition pour administration maternelle comprenant du myo-inositol et de la vitamine B2 et comprenant de la vitamine B6, de la vitamine B12, de la vitamine D, et une combinaison de Lactobacillus et de Bifidobacterium, dans laquelle le Bifidobacterium est la souche de Bifidobacterium lactis BB12 déposée en tant que CNCM I-3446 et le Lactobacillus est la souche de Lactobacillus rhamnosus GG disponible sous le numéro de dépôt CGMCC 1.3724.

2. Composition selon la revendication 1, **caractérisée en ce que** la composition est destinée à être administrée à une femme désirant tomber enceinte, à une femme enceinte ou à une femme qui allaite.

3. Composition selon l'une quelconque des revendications précédentes dans laquelle ladite composition est sous la forme d'une composition nutritionnelle en poudre à reconstituer dans du lait ou de l'eau, un produit alimentaire, une boisson, un complément nutritionnel ou un nutraceutique.

4. Trousse de parties comprenant :

a) les ingrédients myo-inositol, vitamine B2, vitamine B6, vitamine B12, vitamine D, et une combinaison de Lactobacillus et de Bifidobacterium, dans laquelle le Bifidobacterium est la souche de Bifidobacterium lactis BB12 déposée en tant que CNCM I-3446 et le Lactobacillus est la souche de Lactobacillus rhamnosus GG disponible sous le numéro de dépôt CGMCC 1.3724 ; et

b) au moins deux compositions séparées physiquement, comprenant chacune au moins l'un desdits ingrédients, **caractérisée en ce qu'**au moins l'une des compositions séparées physiquement comprend du myo-inositol et au moins l'une des compositions séparées physiquement comprend de la vitamine B2.

**Figure 1**

Micronutrient Deficiency (%)

## Figure 2

A)

6 year percentage fat according to quantiles of maternal serum riboflavin (mean ± SEM , n=475)

4 year percentage fat according to quantiles of maternal serum riboflavin (mean ± SEM , n= 325)

B)

# Figure 3

# Figure 4

**Figure 5**

L6 Glucose uptake
incubation 2H
normal conditions

* p<0.05, ** p<0.01, *** p<0.001

Figure 6

Fasting blood glucose day 18.5

Figure 7

Retroperitoneal fat pads

Figure 8

**Figure 9**

# Figure 10

L6 Glucose uptake
incubation 24H
PALM conditions

Glucose uptake
(pmole/min/mg of proteins)

148%
**

100%

Ctrl_PALM          G -1µM

* p<0.05, ** p<0.01, *** p<0.001

L6 Glucose uptake
incubation 2H
IR conditions

Glucose uptake
(pmole/min/mg of proteins)

160%
**

100%

Ctrl_IR          G -1µM

* p<0.05, ** p<0.01, *** p<0.001

**Figure 11**

delta % fat
(n=20 per group)

**Figure 12**

## fat gain/ weight gain
(n= 20 per group)

**Figure 13**

Insulin pancreatic

**Figure 14**

**AUC insulin increased**
(n=14-19 per group)

**Figure 16**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2014127166 A1
- EP 14161190

### Non-patent literature cited in the description

- **KOLETZKO B et al.** *Am J Nutr,* 2011, vol. 94, 2036-43S
- **TORHEM LE et al.** *J. Nutr.,* 2010, vol. 140, 2051S-58S
- **BAKER L ; PIDDINGTON R.** *J. Diab. Comp.,* 1993, vol. 7, 204-212
- **ATHUKORALA C et al.** *BMC Pregnancy Childbirth,* 2010, vol. 10, 56
- **OVESEN P et al.** *Obstet Gynecol,* 2011, vol. 118, 305
- **AISLING MM.** *Diabetes Care,* 2009, vol. 32 (7), 1308
- Proceedings of the Fourth International Work-shop-Conference on Gestational Diabetes Mellitus. Diabetes Care. 1998, vol. 21, B1-B167
- **CROZE et al.** *Biochimie,* 2013, vol. 95, 1811-1827
- **CLEMENTS RS ; DARNELL B.** *Am J Clin Nutr,* 1980, vol. 33, 1954-1967
- **SCHLEMMER U et al.** *Mol Nutr Food Res,* 2009, vol. 53, S330-S375
- **CANI PD et al.** *Gut,* 2009, vol. 58, 1091-1103